(19) Europäisches Patentamt
European Patent Office
Office européen des brevets

(11) Veröffentlichungsnummer : **0 050 803**
**B1**

(12) # EUROPÄISCHE PATENTSCHRIFT

(45) Veröffentlichungstag der Patentschrift : **13.06.84**

(51) Int. Cl.³ : **A 61 M 1/03**, B 01 D 13/02, C 02 F 1/46

(21) Anmeldenummer : **81108377.3**

(22) Anmeldetag : **15.10.81**

(54) **Verfahren und Vorrichtung zur indirekten Oxidation von Harnstoff.**

(30) Priorität : **27.10.80 DE 3040470**

(43) Veröffentlichungstag der Anmeldung : **05.05.82 Patentblatt 82/18**

(45) Bekanntmachung des Hinweises auf die Patenterteilung : **13.06.84 Patentblatt 84/24**

(84) Benannte Vertragsstaaten : **AT CH DE FR GB LI SE**

(56) Entgegenhaltungen :
**DE-A- 2 261 220**
**FR-A- 2 036 413**
**FR-A- 2 129 165**
**US-A- 3 562 137**
**MEDICAL & BIOLOGICAL ENGINEERING & COMPUTING, Band 16, Nr. 1, Januar 1978, Seiten 25-30 Stevenage, G.B. M. FELS: "Recycle of dialysate from the artificial kidney by electrochemical degradation of waste metabolites: Small-scale laboratory investigations"**

(73) Patentinhaber : **SIEMENS AKTIENGESELLSCHAFT**
**Berlin und München Wittelsbacherplatz 2**
**D-8000 München 2 (DE)**

(72) Erfinder : **Richter, Gerhard, Dr.**
**An der Lauseiche 30**
**D-8520 Erlangen (DE)**
Erfinder : **Weidlich, Erhard, Dr.**
**Am Tennenbach 41**
**D-8520 Erlangen (DE)**
Erfinder : **Mund, Konrad, Dr.**
**Langenbrucker Weg 10**
**D-8521 Uttenreuth (DE)**

**EP 0 050 803 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur indirekten Oxidation von Harnstoff mittels einer zwei Elektroden aufweisenden elektrochemischen Zelle, der eine wäßrige chloridhaltige Harnstofflösung zugeführt wird, sowie eine Vorrichtung zur Durchführung dieses Verfahrens.

Hämodialyse und Hämofiltration sowie Peritonealdialyse sind Verfahren zur Entfernung von Harnstoff aus dem Blut Nierenkranker. Diese Behandlungsmethoden haben im klinischen Bereich bereits einen festen Platz eingenommen und sind zu therapeutischen Routineverfahren geworden. Die Kapazität der vorhandenen Dialysezentren reicht jedoch für die notwendigen Behandlungen der Nierenkranken nicht aus. Hier bietet die Heimdialyse, die vom Patienten selbst durchgeführt werden kann, einen Ausweg. In diesem Fall ist es aber dringend geboten, die für den Patienten erforderlichen Dialyse- und Substitutionslösungen zu verringern. Bisher wird nämlich der bei der extrakorporalen Plasmaregeneration, d. h. bei der Blutreinigung, anfallende harnstoffhaltige Elektrolyt bzw. das Blutfiltrat verworfen, und es muß eine Substitutionslösung bereitgestellt werden. Derzeit sind für die Hämodialyse zwischen 100 und 300 l Dialyselösung und bei der Hämofiltration etwa 20 l Ersatzlösung pro Behandlung erforderlich.

Es sind bereits Versuche unternommen worden, das harnstoffhaltige Dialysat auf elektrochemischem Weg durch indirekte Oxidation des Harnstoffes zu reinigen. Hierzu wird das Dialysat in eine elektrochemische Zelle geleitet, welche in einer Kammer zwei parallel zueinander angeordnete Elektroden aufweist (vgl.: « Medical & Biological Engineering and Computing », Vol. 16, 1978, Seite 25 bis 30). Ein wichtiger Schritt ist hierbei die Bildung von Hypochlorit, das aus dem im Dialysat vorhandenen (Natrium-) Chlorid gebildet wird. An den Elektroden laufen folgende Reaktionen ab :

$$\text{Anode: } 6\ Cl^- \rightarrow 3\ Cl_2 + 6\ e^-;$$

$$\text{Kathode: } 6\ Na^+ + 6\ H_2O + 6\ e^- \rightarrow 6\ NaOH + 3\ H_2.$$

Unter der Voraussetzung, daß eine gute Durchmischung der anodischen und kathodischen Reaktionsprodukte, d. h. Chlor und Natriumhydroxid, erfolgt, bildet sich dann Hypochlorit :

$$6\ NaOH + 3\ Cl_2 \rightarrow 3\ NaOCl + 3\ NaCl + 3\ H_2O.$$

Das gebildete Hypochlorit ist ein starkes Oxidationsmittel, das mit dem Harnstoff in folgender Weise reagiert :

$$(NH_2)_2CO + 3\ NaOCl \rightarrow N_2 + CO_2 + 2\ H_2O + 3\ NaCl.$$

Unter günstigen Bedingungen sollte dabei der Harnstoff also vollständig in Stickstoff, Kohlendioxid und wasser übergeführt werden. In der Praxis hat sich aber gezeigt, daß der Umsatz nicht vollständig ist. Außerdem erfolgt dabei eine $p_H$-Verschiebung und in der behandelten Flüssigkeit bleibt Chlor und Hypochlorit zurück, so daß keine Reinfusion vorgenommen werden kann.

Aufgabe der Erfindung ist es, den bei der Plasmaregeneration anfallenden Elektrolyten, d. h. die wäßbrige chloridhaltige Harnstofflösung, durch indirekte Harnstoffoxidation mittels einer elektrochemischen Zelle derart aufzubereiten, daß der Harnstoff möglichst vollständig entfernt wird, ohne daß sich im Elektrolyten weitere Veränderungen ergeben. Insbesondere soll die Aufbereitung auch in der Weise erfolgen, daß die gereinigte Lösung reinfundiert werden kann.

Dies wird erfindungsgemäß dadurch erreicht, daß die Zelle durch eine ionenleitende Membran in zwei Räume aufgeteilt wird, von einer die Anode und der andere die Kathode enthält, und daß die Lösung zuerst dem Anodenraum und dann dem Kathodenraum zugeführt wird.

Beim erfindungsgemäßen Verfahren wird die aufzubereitende wäßrige Lösung, die unter anderem Harnstoff und Chlorid enthält, zunächst in den Anodenraum der elektrochemischen Zelle geleitet. Dort wird, wie vorstehend bereits erwähnt, durch anodische Oxidation von Chlorid Chlor gebildet :

$$6\ Cl^- \rightarrow 3\ Cl_2 + 6\ e^-.$$

Das Chlor reagiert sofort mit dem Harnstoff und setzt ihn oxidativ zu Stickstoff und Kohlendioxid um :

$$(NH_2)_2CO + 3\ Cl_2 + H_2O \rightarrow N_2 + CO_2 + 6\ H^+ + 6\ Cl^-.$$

Im Anodenraum sinkt also — infolge der Bildung von Wasserstoffionen — der $p_H$-Wert des Elektrolyten.

Vom Anodenraum aus gelangt der weitgehend harnstofffreie Elektrolyt in den Kathodenraum, wo eine kathodische Wasserstoffentwicklung erfolgt :

$$6\ H^+ + 6\ e^- \rightarrow 3\ H_2.$$

Infolge des Verbrauchs an Wasserstoffionen und des Transports von Natriumionen durch die Membran vom Anoden- in den Kathodenraum steigt dabei der $p_H$-Wert wieder an und die Lösung wird weitgehend neutral. Die Gesamtreaktion dieser elektrochemischen Harnstöffoxidation, bei der der Harnstoff nicht direkt durch elektrochemische Oxidation an der Anode entfernt wird, sondern indirekt durch Oxidation mit anodisch entwickeltem Chlor, ergibt sich zu :

$$(NH_2)_2CO + H_2O \rightarrow N_2 + CO_2 + 3\ H_2.$$

Beim erfindungsgemäßen Verfahren wird die zu behandelnde Lösung weitesgehend vom Harnstoff befreit, ohne daß schädliche Nebenprodukte anfallen. Diese Lösung kann somit reinfundiert werden. Dabei ergibt sich der Vorteil, daß bei der (extrakorporalen) Blutreinigung keine zusätzlichen Flüssigkeiten, wie Dialyse- und Ersatzlösungen, benötigt werden. Im Vergleich zu bisher verwendeten Systemen zur Blutreinigung ergibt sich beim Einsatz des erfindungsgemäßen Verfahrens ferner eine Vereinfachung im Aufbau sowie eine Verkleinerung und darüber hinaus auch eine Verbilligung.

Die beim erfindungsgemäßen Verfahren eingesetzte Membran ist ionenleitend. Darüber hinaus soll diese Membran relativ dicht sein, um eine Diffusion von Harnstoff weitgehend zu unterbinden. Bei einer besonders bevorzugten Vorrichtung zur Durchführung des erfindungsgemäßen Verfahrens, d. h. bei einer elektrochemischen Zelle zur indirekten Harnstoffabreicherung, sind der Anoden- und der Kathodenraum (der elektrochemischen Zelle) durch eine Kationenaustauschermembran voneinander getrennt. Diese Membran soll nämlich insbesondere für Natriumionen durchlässig sein. In diesem Fall ist es auch vorteilhaft, wenn der Strömungswiderstand der Membran groß ist gegenüber demjenigen der Elektrolyträume, d. h. von Anoden- und Kathodenraum. Es hat sich dabei als besonders vorteilhaft erwiesen, wenn zur Trennung der Elektrolyträume eine Membran aus sulfoniertem Polytetrafluoräthylen dient. Eine derartige Membran ermöglicht nämlich nicht nur den Durchtritt von Natriumionen, sondern sie ist darüber hinaus gegenüber Chlor vollkommen inert.

Bei der erfindungsgemäßen Vorrichtung gemäß Anspruch 2 ist es ferner von Vorteil, wenn die Elektroden porös sind und die Elektrolyträume im wesentlichen vollständig ausfüllen, d. h. wenn Anode und Kathode sich unmittelbar bis zu der sie trennenden Membran erstrecken. Auf diese Weise verbleiben im wesentlichen keine freien Elektrolyträume, vielmehr übernehmen die porösen Elektrodenstrukturen quasi deren Funktion. Hierbei ergibt sich der Vorteil, daß die Elektrolytflüssigkeit ständig umgeleitet wird, so daß eine für eine weitgehende Oxidation des Harnstoffes günstige Verwirbelung erfolgt. Gleichzeitig wird hierdurch gewährleistet, daß im Anodenraum gebildetes überschüssiges Chlor im Kathodenraum wieder vollständig reduziert wird.

Weitere vorteilhafte Ausführungsformen der erfindungsgemäßen Vorrichtung sind Gegenstand von abhängigen Ansprüchen.

Anhand von Ausführungsbeispielen und Figuren soll die Erfindung noch näher erläutert werden.

In Fig. 1 ist — unter Zugrundelegung einer schematisch dargestellten elektrochemischen Zelle — das Prinzip der beim erfindungsgemäßen Verfahren stattfindenden indirekten elektrochemischen Harnstoffoxidation wiedergegeben. Wie vorstehend dargelegt, erfolgt dabei an der Anode eine Chlorentwicklung (durch Oxidation von Chlorid) ; im Anolyten bewirkt dieses Chlor dann die Oxidation des Harnstoffes und an der Kathode werden schließlich die bei der Harnstoffoxidation gebildeten Wasserstoffionen — unter Freisetzung von Wasserstoffreduziert.

In Fig. 2 ist — in einer Art « exploded view » — der Aufbau der elektrochemischen Zelle 10 einer bevorzugten Ausführungsform der erfindungsgemäßen Vorrichtung im Schnitt dargestellt. In der Aussparung einer Halterung 11, beispielsweise aus Polymethacrylat, ist eine poröse Anode 12 angeordnet und entsprechend in einer Halterung 13 eine poröse Kathode 14. Die zu behandelnde Lösung 13 eine poröse Kathode 14. Die zu behandelnde Lösung wird über eine Leitung 15 dem Anodenraum 16 zugeführt. Eine Leitung 17 gestattet den Übertritt der Lösung vom Anodenraum 16 in den Kathodenraum 18, aus dem die Lösung durch eine Leitung 19 entfernt wird. Zwischen den Halterungen 11 und 13 ist eine ionenleitende Membran 20 angeordnet. Im betriebsbereiten Zustand liegen die Elektroden 12 und 14 direkt an der Membran 20 an. Die elektrischen Kontakte der beiden Elektroden sind in Fig. 2 nicht dargestellt.

Die Anode besteht bei der erfindungsgemäßen Vorrichtung vorzugsweise aus aktiviertem Titan, insbesondere in Form eines Netzes oder Streckmetalles. Ferner kann die Anode aus einem Platinblech mit einem oder mehreren aufgepunkteten Netzen aus Platin oder aus Platin und Iridium bestehen. Anstelle der Netze können auch Streckmetalle aus Titan oder Tantal eingesetzt werden, die mit Platinmohr oder Platin und Rutheniumoxid belegt sind. Vorteilhaft ist es auch, gegen Chlor beständige poröse Metalle, wie Raney-Platin und porösen Titanschwamm, oder gesinterte Metalle, die gegebenenfalls mit Edelmetallkatalysatoren belegt sind, als Elektrodenmaterial einzusetzen.

Die Kathode besteht vorzugsweise aus einem aktivierten Kohlefaservlies. Ferner kann die Kathode aus einem Platinblech, einem Platinnetz, einer Glaskohlenstoffplatte oder einem Kohlefilz bestehen. Der Kohlefilz kann — ebenso wie das Kohlefaservlies — aktiviert sein. Die Aktivierung erfolgt dabei vorzugsweise durch Erhitzen an Luft bei Temperaturen zwischen 200 und 700 °C, vorzugsweise etwa bei 500 °C, oder in Kohlendioxid bei Temperaturen zwischen 500 und 1 200 °C, vorzugsweise etwa bei 1 000 °C, und/oder durch Belegen mit Platin oder einem anderen Edelmetallkatalysator.

Im Betrieb durchströmt das Blutfiltrat zunächst den Anodenraum. Dort erfolgt die Chlorentwicklung und es findet die Oxidation des Harnstoffes statt. Dabei sinkt der $p_H$-Wert der ursprünglich annähernd neutralen Lösung auf Werte etwa zwischen 1,3 und 1,6 ab, d. h. die Lösung reagiert sauer. Vom Anodenraum aus gelangt das Reaktionsgemisch in den Kathodenraum. Dort wird das überschüssige Chlor reduziert und gleichzeitig steigt der $p_H$-Wert der Lösung — infolge der kathodischen Reduktion von Wasserstoffionen — wieder auf Werte etwa zwischen 6,5 und 7,8 an, d. h. die Lösung wird annähernd neutral.

Durch eine Reaktionsführung der vorstehend genannten Art läßt sich der Harnstoff beispielsweise bei einer Ausgangskonzentration von 2 g/l bis auf etwa 100 mg/l abreichern, d. h. die Abreicherungsrate beträgt bis zu 95 %. Die Harnstoffoxidation ist dabei abhängig von der Stromstärke und der Durchlaufgeschwindigkeit. So ergeben sich die obengenannten Werte bei einer Stromstärke von 200 $mA/cm^2$ (Elektrodengröße : 60 $cm^2$ ; Temperatur : 20 °C). Bei einer Stromstärke von 100 $mA/cm^2$ (Temperatur : 37 °C) erreicht die Faraday-Ausbeute $\gamma$ bei verschiedenen Durchlaufgeschwindigkeiten beispielsweise etwa folgende Werte : 5 $cm^3/min$ : 0,3 ; 10 $cm^3/min$ : 0,45 ; 20 $cm^3/min$ : 0,55.

Um die Effektivität der erfindungsgemäßen Vorrichtung zu erhöhen, ist es vorteilhaft, mehrere elektrochemische Zellen in Reihe zu schalten. Bei einer dreistufigen Anordnung beispielsweise kann der Harnstoffgehalt — bei einer Stromstärke von 12 A und einer Strömungsgeschwindigkeit von 60 ml/min — von 200 mg/dl auf 3 mg/dl verringert werden, bei einem Faraday-Wirkungsgrad über 50 %. Innerhalb einer Stunde können mit einer derartigen Anordnung 7 g Harnstoff eliminiert werden und somit etwa in 3 Stunden der im Zeitraum von einem Tag anfallende Harnstoff.

Ferner kann bei der erfindungsgemäßen Vorrichtung auch eine Thermostatisierung der elektrochemischen Zelle bzw. Zellen vorgesehen sein. Dazu werden die Elektrolyträume auf den von der Membran abgewandten Seiten der Elektroden beispielsweise durch Trennbleche aus Titan abgeschlossen, wobei Räume gebildet werden, die an einen Thermostaten angeschlossen werden können.

Bei der erfindungsgemäßen Vorrichtung kann es auch vorteilhaft sein, der elektrochemischen Zelle bzw. deren Kathodenraum strömungsmäßig einen oder mehrere Reaktoren zur Beseitigung von Spuren an Hypochlorit nachzuschalten. Dabei ist der Reaktor vorzugsweise mit der Kathode der elektrochemischen Zelle elektrisch leitend verbunden, so daß eine kathodische Reduktion von Hypochlorit erfolgt.

Der Reaktor besteht vorzugsweise aus einem Rohr, insbesondere aus Polymethacrylat, das an der Wandung mit einem Metallnetz ausgelegt und mit einem elektrischen Anschluß versehen ist ; das Rohr selbst ist mit Aktivkohle gefüllt. Die Aktivkohle kann, wie die Kohlegewebe in der Kathode der elektrochemischen Zelle, aktiviert und/oder mit Platin oder einem anderen Edelmetallkatalysator belegt sein. Vorteilhaft wird im Reaktor platinierte Kohle eingesetzt. In diesem Fall wird nämlich an der platinierten Kohle das Wasserstoffpotential eingestellt, weil der den Kathodenraum der elektrochemischen Zelle verlassende Elektrolyt Wasserstoff in gelöster Form enthält. Auf diese Weise können dann die Reste an Hypochlorit — und gegebenenfalls auch an Chlor — sehr leicht entfernt werden.

In Fig. 3 ist schematisch ein Kreislauf zur Regeneration von Blutfiltrat dargestellt, wobei eine bevorzugte Ausführungsform der erfindungsgemäßen Vorrichtung Anwendung findet. Dabei wird einer Körperarterie kontinuierlich Blut entnommen, das mittels einer Blutpumpe 30 einem Ultrafilter 31 zugeführt wird, in welchem eine Hämofiltration erfolgt. Vom Ultrafilter 31 gelangt das restliche blut über eine Blasenfalle 32 zurück in den Körper, wobei es einer Vene zugeführt wird. Vor der Hämofiltration wird dem Blut mittels einer Einrichtung 33 noch Heparin zugesetzt.

Das im Ultrafilter 31 anfallende Blutfiltrat wird durch ein Kohlefilter 34 geleitet und dann der elektrochemischen Zelle 35 zugeführt, und zwar dem Anodenraum 36. Der Anodenraum 36, der die Anode 37 enthält, ist vom Kathodenraum 38 mit der Kathode 39 durch eine Membran 40 getrennt. Das Bluttfiltrat, d. h. der Elektrolyt, tritt durch eine Leitung 41 vom Anodenraum 36 in den Kathodenraum 38 über und gelangt nach dem Austritt aus dem Kathodenraum in einen Reaktor 42. Der Reaktor 42 weist eine Elektrode 43 auf, welche mit der Kathode 39 der elektrochemischen Zelle 35 elektrisch leitend verbunden ist. Dem Reaktor 42 ist strömungsmäßig vorteilhaft ein Kohlefilter 44 nachgeschaltet, von welchem aus das gereignite Blutfiltrat — über die Blasenfalle 32 — in den Blutkreislauf zurückgeleitet wird. Zur Förderung des Bluttfiltrates kann eine Pumpe vorgesehen werden, die beispielsweise zwischen dem Filter 34 und der elektrochemischen Zelle 35 angeordnet wird.

Bei einem Vergleich der bei Durchführung des erfindungsgemäßen Verfahrens erhaltenen Ergebnisse mit denjenigen des bekannten Verfahrens, das ohne Trennung von Anoden- und Kathodenraum arbeitet, zeigen sich erhebliche Unterschiede. So können beim bekannten Verfahren nur ca. 70 % des Harnstoffes umgesetzt werden, während beim erfindungsgemäßen Verfahren die Umsetzung nahezu quantitativ erfolgt. Beim bekannten Verfahren sinkt ferner der $p_H$-Wert des Elektrolyten während der Harnstoffabreicherung, d. h. der Elektrolyt wird sauer, und darüber hinaus bleiben hierbei Reste von Chlor und Hypochlorit im Elektrolyten zurück, so daß eine Reinfusion nicht möglich ist, weil dies nicht zu vertreten wäre. Diese Nachteile werden beim erfindungsgemäßen Verfahren bzw. der entsprechenden Vorrichtung durch Aufteilung des Elektrolytraumes in einen Anoden- und einen Kathodenraum vermieden, wobei im Anodenraum zunächst der Harnstoff umgesetzt und somit entfernt wird, und dann im Kathodenraum der Elektrolyt — mit Ausnahme des Harnstoffes — wieder ins Gleichgewicht gesetzt wird, d. h. die ursprünglichen Verhältnisse wieder hergestellt werden, so daß eine Reinfusion vorgenommen werden kann.

**Ansprüche**

1. Verfahren zur indirekten Oxidation von Harnstoff mittels einer zwei Elektroden (12, 14) aufweisenden elektrochemischen Zelle (10), der eine wäßrige chloridhaltige Harnstofflösung zugeführt wird, dadurch gekennzeichnet, daß die Zelle (10) durch eine ionenleitende Membran (20) in zwei Räume (16, 18) aufgeteilt wird, von denen einer die Anode (12) und der andere die Kathode (14) enthält, und daß die Lösung zuerst dem Anodenraum (16) und dann dem Kathodenraum (18) zugeführt wird.

2. Vorrichtung zur Durchführung des Verfahrens nach Anspruch 1, mit einer zwei Elektroden (12, 14) aufweisenden elektrochemischen Zelle (10), dadurch gekennzeichnet, daß Anoden- und Kathodenraum (16, 18) der elektrochemischen Zelle (10) durch eine Kationenaustauschermembran (20) voneinander getrennt und durch eine überführungsleitung (17) miteinander verbunden sind.

3. Vorrichtung nach Anspruch 2, dadurch gekennzeichnet, daß der Strömungswiderstand der Membran (20) groß ist gegenüber demjenigen von Anoden- und Kathodenraum (16, 18).

4. Vorrichtung nach Anspruch 2 oder 3, dadurch gekennzeichnet, daß die Membran (20) aus sulfoniertem Polytetrafluoräthylen besteht.

5. Vorrichtung nach einem der Ansprüche 2 bis 4, dadurch gekennzeichnet, daß die Elektroden (12, 14) porös sind und den Anoden- bzw. Kathodenraum (16, 18) ausfüllen.

6. Vorrichtung nach einem oder mehreren der Ansprüche 2 bis 5, dadurch gekennzeichnet, daß die Anode (12) aus aktiviertem Titan besteht, vorzugsweise in Form eines Netzes oder Streckmetalles.

7. Vorrichtung nach einem oder mehreren der Ansprüche 2 bis 6, dadurch gekennzeichnet, daß die Kathode (14) aus einem aktivierten Kohlefaservlies besteht.

8. Vorrichtung nach Anspruch 6 oder 7, dadurch gekennzeichnet, daß die Elektroden (12, 14) mit Edelmetallen aktiviert sind.

9. Vorrichtung nach einem oder mehreren der Ansprüche 2 bis 5, dadurch gekennzeichnet, daß die Elektroden (12, 14) Elektrokatalysatoren enthalten.

10. Vorrichtung nach einem oder mehreren der Ansprüche 2 bis 9, dadurch gekennzeichnet, daß der elektrochemischen Zelle (10) wenigstens ein Reaktor (42) zur Beseitigung von Hypochloritresten nachgeschaltet ist und daß der Reaktor (42, 43) vorzugsweise mit der Zelle (10) elektrisch leitend verbunden ist.

11. Vorrichtung nach Anspruch 10, dadruch gekennzeichnet, daß der Reaktor (42) aus einem Rohr besteht, das an der Wandung mit einem Metallnetz ausgelegt ist, daß das Metallnetz mit einem elektrischen Anschluß versehen ist und daß das Rohr mit Aktivkohle gefüllt ist.

12. Vorrichtung nach Anspruch 11, dadurch gekennzeichnet, daß die Aktivkohle aktiviert und/oder mit Edelmetallkatalysatoren belegt ist.

13. Vorrichtung nach einem der Ansprüche 10 bis 12, dadurch gekennzeichnet, daß dem Reaktor (42) ein Kohlefilter (44) nachgeschaltet ist.

14. Vorrichtung nach einem oder mehreren der Ansprüche 2 bis 13, dadurch gekennzeichnet, daß mehrere elektrochemische Zellen (10) hintereinander angeordnet sind.

**Claims**

1. A process for the indirect oxidation of urea by means of an electro-chemical cell (10) which has two electrodes (12, 14) and to which an aqueous chloride-containing urea solution is fed, characterised in that the cell (10) is divided into two chambers (16, 18) by an ion-conducting membrane (20), one of which chambers contains the anode (12) and the other of which contains the cathode (14) ; and that the solution is first fed into the anode chamber (16) and subsequently into the cathode chamber (18).

2. Apparatus for carrying out the process according to Claim 1, with an electro-chemical cell (10) which has two electrodes (12, 14), characterised in that anode and cathode chambers (16, 18) of the electro-chemical cell (10) are separated from one another by a cation exchange membrane (20) and are connected to one another by a transfer line (17).

3. Apparatus according to Claim 2, characterised in that the flow resistance of the membrane (20) is large compared with that of the anode and cathode chambers (16, 18).

4. Apparatus according to Claim 2 or Claim 3, characterised in that the membrane (20) consists of sulphonated polytetrafluoroethylene.

5. Apparatus according to one of Claims 2 to 4, characterised in that the electrodes (12, 14) are porous and fill the anode and cathode chambers (16, 18).

6. Apparatus according to one or more of Claims 2 to 5, characterised in that the anode (12) consists of activated titanium, preferably in the form of a network or expanded metal.

7. Apparatus according to one or more of Claims 2 to 6, characterised in that the cathode (14) consists of an activated fibrous carbon fleece.

8. Apparatus according to Claim 6 or Claim 7, characterised in that the electrodes (12, 14) are activated with noble metals.

9. Apparatus according to one or more of Claims 2 to 5, characterised in that the electrodes (12, 14) contain electro-catalysts.

10. Apparatus according to one or more of Claims 2 to 9, characterised in that the electro-chemical cell (10) is followed by at least one reactor (42) for removing hypochlorite residues ; and that the reactor (42, 43) is preferably connected to the cell (10) in electrically conductive manner.

11. Apparatus according to Claim 10, characterised in that the reactor (42) consists of a tube which is lined with a metal network at the wall ; that the metal network is provided with an electric terminal ; and that the tube is filled with activated carbon.

12. Apparatus according to Claim 11, characterised in that the activated carbon is activated and/or coated with noble metal catalysts.

13. Apparatus according to one of Claims 10 to 12, characterised in that the reactor (42) is followed by a carbon filter (44).

14. Apparatus according to one or more of Claims 2 to 13, characterised in that a plurality of electrochemical cells (10) are arranged in succession.

## Revendications

1. Procédé d'oxydation indirecte de l'urée au moyen d'une cellule électrochimique (10) qui présente deux électrodes (12, 14) et à laquelle est amenée une solution aqueuse d'urée contenant du chlorure, caractérisé en ce qu'il consiste à subdiviser la cellule (10) par une membrane (20) conduisant les ions, en deux compartiments (16, 18) dont l'un contient l'anode (12) et l'autre la cathode (14), et à envoyer la solution d'abord au compartiment anodique (16) et ensuite au compartiment cathodique (18).

2. Appareil pour exécuter le procédé suivant la revendication 1, comprenant une cellule électrochimique (10) présentant deux électrodes (12, 14), caractérisé en ce que les compartiments anodique et cathodique (16, 18) de la cellule électrochimique (10) sont séparés l'un de l'autre par une membrane échangeuse de cations (20) et communiquent l'un avec l'autre par un conduit de passage (17).

3. Appareil suivant la revendication 2, caractérisé en ce que la résistance à l'écoulement de la membrane (20) est grande par rapport à celle du compartiment anodique et cathodique (16, 18).

4. Appareil suivant la revendication 2 ou 3, caractérisé en ce que la membrane (20) est en polytétrafluoroéthylène sulfoné.

5. Appareil suivant l'une des revendications 2 à 4, caractérisé en ce que les électrodes (12, 14) sont poreuses et emplissent le compartiment anodique et cathodique (16, 18).

6. Appareil suivant l'une ou plusieurs des revendications 2 à 5, caractérisé en ce que l'anode (12) est en titane activé, de préférence sous forme d'une grille ou d'un métal déployé.

7. Appareil suivant l'une ou plusieurs des revendications 2 à 6, caractérisé en ce que la cathode (14) est en tissu non tissé en fibres de carbone activé.

8. Appareil suivant la revendication 6 ou 7, caractérisé en ce que les électrodes (12, 14) sont activées par des métaux nobles.

9. Appareil suivant l'une ou plusieurs des revendications 2 à 5, caractérisé en ce que les électrodes (12, 14) contiennent des électrocatalyseurs.

10. Appareil suivant l'une ou plusieurs des revendications 2 à 9, caractérisé en ce qu'au moins un réacteur (42) pour la suppression des restes d'hypochlorite est monté en aval de la cellule électrochimique (10) et en ce que le réacteur (42, 43) est relié avantageusement à la cellule (10) d'une manière conductrice de l'électricité.

11. Appareil suivant la revendication 10, caractérisé en ce que le réacteur (42) est constitué d'un tube à parois garnies d'une grille métallique, en ce que la grille métallique est munie d'une conexion électrique et en ce que le tube est empli de charbon actif.

12. Appareil suivant la revendication 11, caractérisé en ce que le charbon actif est activé et/ou est revêtu de métaux nobles servant de catalyseur.

13. Appareil suivant l'une des revendications 10 à 12, caractérisé en ce qu'un filtre en charbon (44) est monté en aval du réacteur (42).

14. Appareil suivant l'une ou plusieurs des revendications 2 à 13, caractérisé en ce que plusieurs cellules électrochimiques (10) sont montées en série.

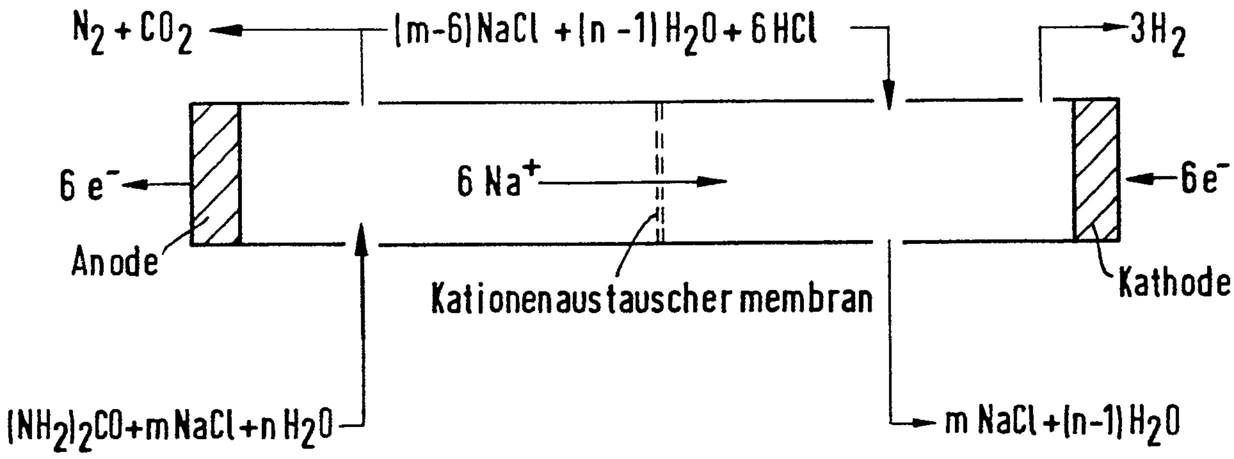
$N_2 + CO_2$
$(m-6) NaCl + (n-1) H_2O + 6 HCl$
$3H_2$
$6 e^-$
$6 Na^+$
$6e^-$
Anode
Kationenaustauschermembran
Kathode
$(NH_2)_2CO + m NaCl + n H_2O$
$m NaCl + (n-1) H_2O$

FIG 1

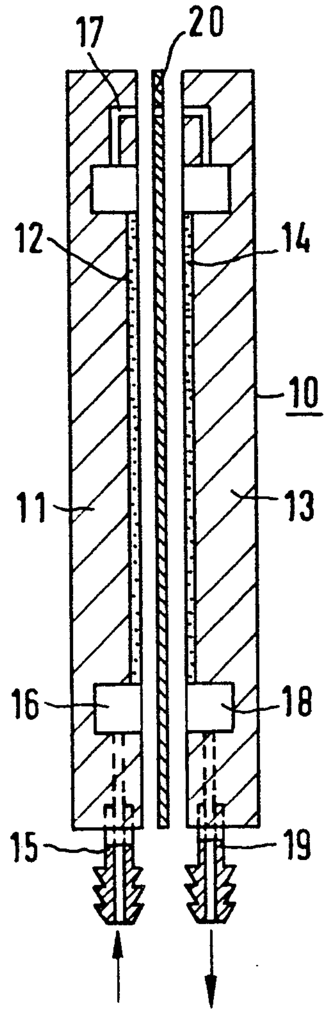
17
20
12
14
10
11
13
16
18
15
19

FIG 2

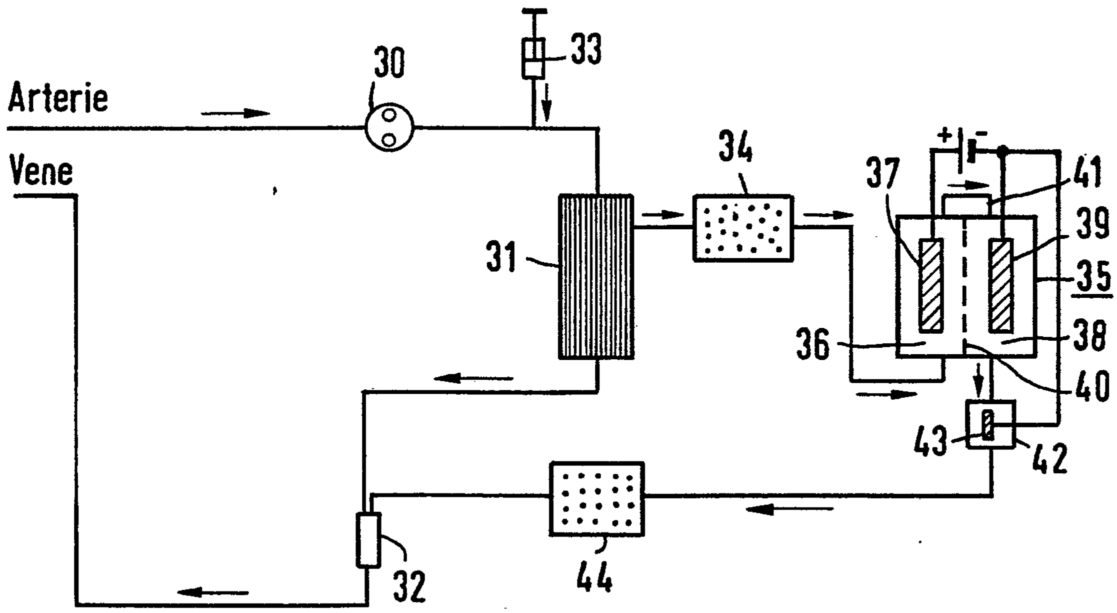


FIG 3